# EUROPEAN PATENT SPECIFICATION

(11) **EP 0 588 856 B1**
(45) Date of publication and mention of the grant of the patent: **14.01.1998**
(21) Application number: 92911563.2
(22) Date of filing: 27.05.1992
(51) Int. Cl.: A61M 39/02, A61M 39/10

(54) **CONNECTING DEVICE FOR MEDICAL PURPOSES**
VERBINDUNGSEINRICHTUNG FÜR MEDIZINISCHE ZWECKE
DISPOSITIF DE BRANCHEMENT UTILISE EN MEDECINE

(30) Priority: 29.05.1991 SE 9101631
(43) Date of publication of application: 30.03.1994
(73) Proprietor: MAGNUSSON, Tore, S-741 00 Knivsta (SE); ORBAN, Laszlo, 199 91 Enköping (SE); DANIELSON, Bo G., S-756 45 Uppsala (SE); PETERSSON, Torgny, 740 63 Österbybruk (SE)
(72) Inventor: MAGNUSSON, Tore, S-741 00 Knivsta (SE); ORBAN, Laszlo, 199 91 Enköping (SE); DANIELSON, Bo G., S-756 45 Uppsala (SE); PETERSSON, Torgny, 740 63 Österbybruk (SE)
(74) Representative: Modin, Jan
(86) International application number: SE9200358
(87) International publication number: WO9221403

(56) References cited:
- FR-A- 1 515 030
- SE-B- 464 665
- US-A- 953 129

## Description

This invention concerns a connecting device according to the preamble of claim 1.

When handling fluids for medical purposes there is often a need of connecting a fluid conduit to one or more other conduits. One example of this is when a bag reservoir containing a nutrition solution or the like shall be connected to the blood stream of a patient. In a connecting device of this kind, there is a risk of leakage from and between the connecting parts at the same time as no indication is given that connection has been achieved in a proper manner. A further problem with previously known conduit connecting devices is the relatively high flow resistance that they are sometimes associated with.

Through EP-A1-0 346 785 a connecting device is previously known, where the valve function is situated in the respective connecting half. This known device is complex in its construction and contains valve bodies which are cam operated and spring actuated. Due to this construction there is a great risk of making mistakes when connecting, because a number of different angular positions also correspond to different functions of the valve bodies. The device is further associated with a relatively high flow resistance.

FR-A- 1 515 030 concerns a connecting device for fluids, which comprises a connecting housing, a valve body and a connecting member. The valve body is operated by connecting the connecting member. There are as a fact several connection possibilities present. This connecting device is however complex in its construction and apparently calls for a high grade of precision in assembling as well as in use. The device thus has several sealing rings for sealing between on the one hand the valve body and on the other hand the coupling housing as well as the connecting member. Further, the valve body is inserted within the coupling housing and stationary within this by means of a cover, which is screwed onto the upper part of the housing.

The applicant's own SE-B-464 665 concerns a connecting device of the above mentioned kind where, however, sealing rings are necessary for sealing between the different movable parts of the device. The valve body is fixed axially within the coupling housing by means of a central pin, which penetrates the wall of the coupling housing and is provided with a thickening at the outside thereof. Also this lead through is provided with a sealing ring. In an alternative embodiment, the valve body is axially fixed by means of a so-called spring lock, which penetrates a grove in the coupling housing as well as in the valve body.

All the above mentioned previously known devices are associated with different draw back concerning their construction as to manufacturing and handling in use.

It is an aim of the invention to achieve a connecting device for medical purposes which solves the above problems, is of simple construction, and the different parts of which are possible to manufacture with high precision from a synthetic material, has a relatively low flow resistance, safe valve function and gives a safe indication that the different connecting members are connected in a proper manner. These aims are achieved by the invention characterized by the features of the characterizing part of claim 1. Several advantages are achieved with this construction of the connecting device. By the valve body having a ring shaped integral seat surface for direct sealed connection to a lower surface of an integral sealing nipple on the connecting member, smooth conduit connection is achieved between the connecting member and the valve body without steps or ring shaped groves there between. This has proved to be a great advantage in handling different fluids for medical purposes, including blood. By the connecting device according to the invention being free from separate sealing elements, and by the valve body having integral fastening projections for snap-in cooperation with snap recesses in the coupling housing, a minimizing of the number of elements in the connecting device is achieved, as well as possibility of a very safe and simple mounting of the valve body within the coupling housing. By its character of a unified system where the different units are mutually compatible, great handling advantages are also achieved. When for instance the bag reservoir connection may be achieved simply and without leakage with a septum unit (claim 5) great flexibility is achieved.

The features of claims 10 and 11 has proved to bring about particularly great advantages in connection with the fastening of the valve body within the coupling housing, at the same time as the designated elements are easily manufactured from a synthetic material.

Further advantages of the invention are achieved by the features of the other dependent claims.

The invention will now be described in greater detail in connection with the annexed drawings, on which:
Fig.1 is an assembled section through a first embodiment of the invention in a connected and open position;
Fig. 2 is an assembled section through the first embodiment of the invention with the connecting member in an inserted position and the valve in a closed position;
Figs. 3a - c show the embodiment according to Figs. 1 and 2 in an exploded perspective view;
Fig. 4 show the first embodiment in an application with an alternative connecting member;
Figs. 5a - c show the parts of the connecting member of Fig. 4 in an exploded perspective view;
Fig. 6 is a sectioned perspective view of a second embodiment of the invention in a connected and open position;
Figs. 7 and 8 are assembled perspective views of a third embodiment in a connected and open position and an inserted but closed position respectively;
Figs. 9a - c show the device according to Figs. 7 and 8 in an exploded perspective view; and
Fig. 10 is a perspective view of the device according to Fig. 9c.

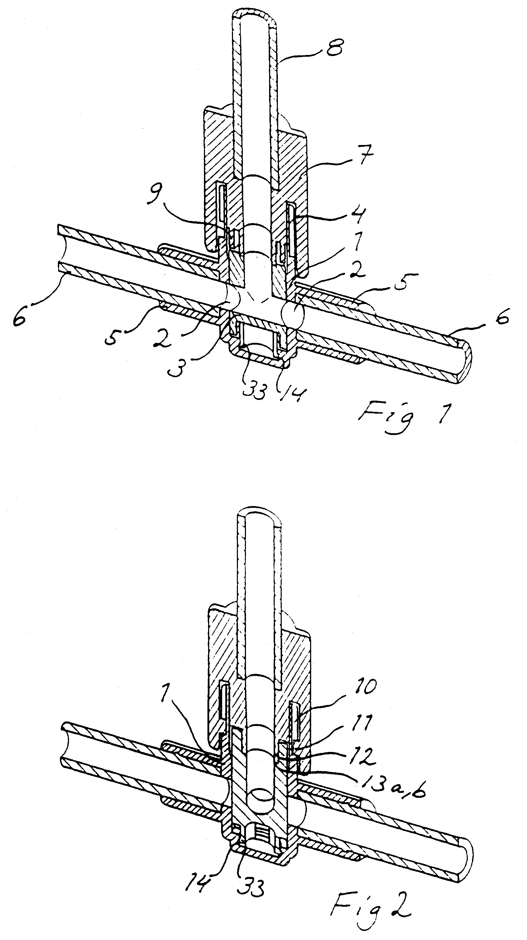

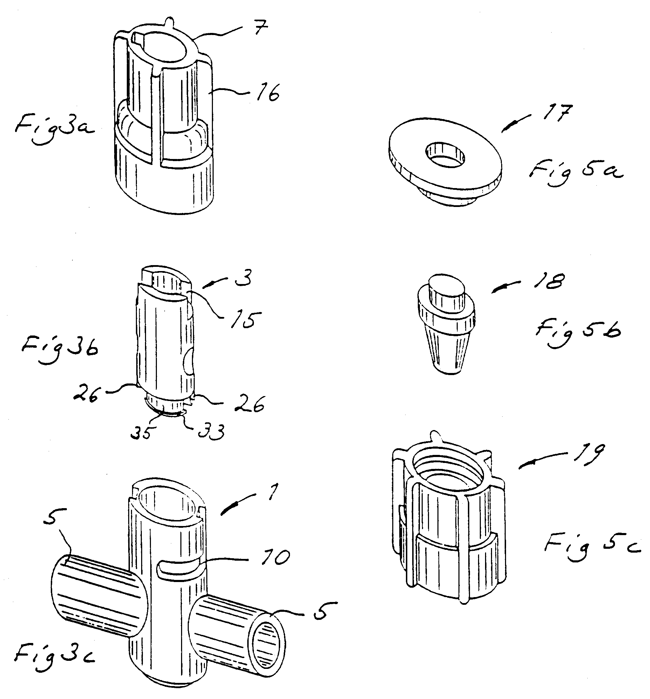

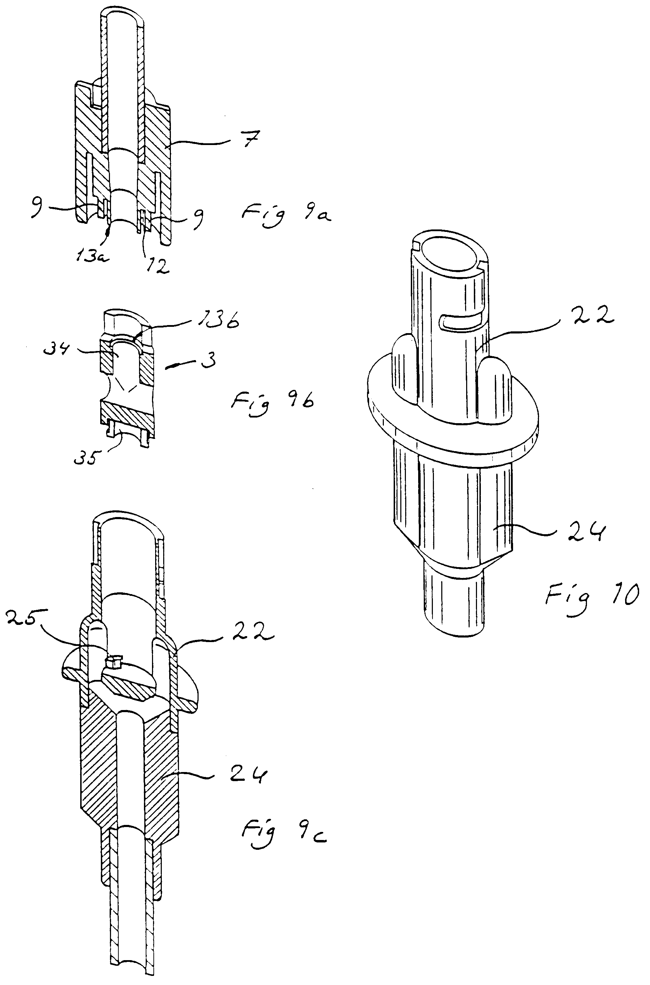

In Fig. 1 a coupling housing 1 comprises two circuit connectors 2, which via circuit nipples 5 connect the coupling housing 1 with a circuit being formed by a circuit house 6, through which e.g. blood is brought to flow. The circuit Ripples 5 form seats for the circuit house 6, so that the inner diameter of the hose corresponds to the diameter of the circuit connector. A valve body 3 is rotatable within the coupling housing but axially fixed therein with snapping action by means of a fastening lip 33 in cooperation with a fastening grove 14 within the housing. The central portion of the coupling housing 1 has an essentially circular cylindric shape and comprises at its upper part (in the figure) an inlet portion 4, which at the outside is provided with joining groves 10 for the cooperation with connecting bosses 11 of a connecting unit 7, which is intended to connect the coupling housing with a connecting hose 8. The valve body has an inverted T-shaped conduit configuration, where the horizontal portion cooperates with the circuit connectors 2 and the verticle portion with the connecting unit 7. For that purpose the connecting unit 7 at its lower inner portion is provided with a sealing nipple 12, the lower (in the Fig.) horizontal surface of which (13a in Fig. 9a) cooperates with a surface (13b in Fig. 9b) on the valve body 3 for achieving a sealed connection. The connecting unit 7 cooperates with the valve body 3 as to rotation by engaging means 9 which engage in engaging recesses 15 in the valve body 3 (Fig. 3b). The valve body 3 is further directly inserted within the coupling housing 1 without separate sealing elements.

When joining a connecting hose 8 with the circuit hose 6 in the embodiment of the connecting device shown in Figs. 1 and 2, the connecting unit 7 is firstly inserted into the position shown in Fig. 2, whereby the engaging means 9 engage the engaging recesses 15 (Fig. 3b). Then the connecting unit 7 is turned an angle of 90°, whereby the connecting unit 7 is coupled together with the coupling housing 1 at the same time as the valve body 3 is turned to the position shown in Fig. 1, with open circuit connectors and the connecting hose 8 joined with the circuit hose 6.

Fig. 3a shows the connecting unit 7 in a perspective view with four turning wings 16. Fig. 3b shows the valve body 3 with engaging recesses 15, which are comprised of a transverse cut in the upper part of the valve body. This figure also shows the radially outwards directed fastening flange 33, which is formed integral with the rest of the valve body on an axially downwards projecting partly cylindrical sleeve shaped portion 35, with a smaller radius than that of the envelope surface of the valve body 3. This embodiment of the fastening means for the valve body brings about great simplicity regarding assembling and manufacturing as well as great reliability. Preferably the valve body is provided with two sleeve shaped portions 35 situated opposite each other for engagement into the fastening grove 14 which is formed within the housing. Fig 3c shows, also in a perspective view, the coupling housing 1 with circuit nipples 5 and joining groves 10.

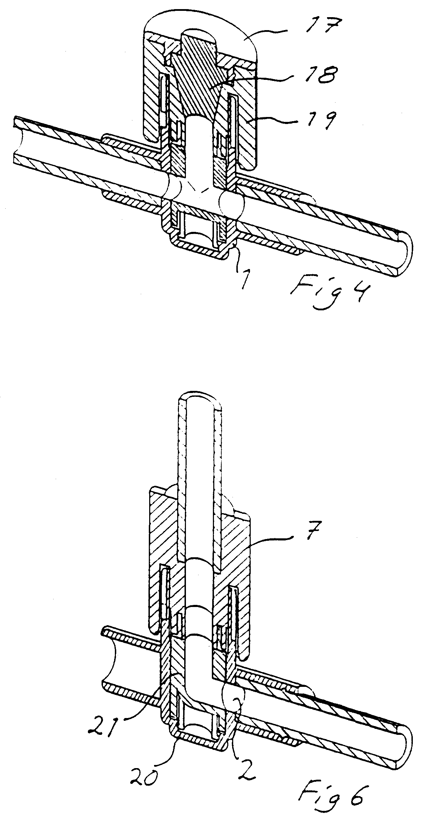

In the variant of the first embodiment shown in Fig. 4, a septum unit 19 is coupled into the inlet portion of the coupling housing. The septum unit 19 forms, together with a cover 17, which is fixed to the septum unit 19 by means of a grove fastening, a space for a penetratable septum 18 of e.g. silicone rubber. The joining of the septum unit 19 with the coupling housing 1 is achieved in the same way as was already described for the connecting unit 7. Joining of the septum unit 19 gives possibility of introducing different fluids containing e.g. medicine by means of an injection needle.

The Figs. 5a, b and c show the cover 17, the septum 18 and the septum unit 19 respectively in perspective views.

Fig. 6 shows a second embodiment of the invention with a modified coupling housing 20 with one single circuit connector 2 and a modified valve body 21 with L-shaped connecting channel, where the horizontal portion (in the figure) cooperates with the circuit connector 2 and the vertical portion (in the figure) cooperates with the connecting unit 7 in a manner already described above. The connecting device according to Fig. 6 is used when there is a demand for a coupling housing with only one circuit connector 2.

Fig. 7 shows a third embodiment of the invention where the connecting unit 7 is joined with a coupling housing 22 containing the valve body 3 in a previously described manner. The coupling housing 22 comprises peripheral axial channels 23, which via a funnel shaped portion connects the horizontal channel portion of the valve body 3 with a central channel of a hose connecting portion 24, which is integral with the lower part (in the Fig.) of the coupling housing 22. The coupling housing 22 has an outward protruding flange at its lower part for stiffening purposes. The flange also serves as a support when handling the coupling device. The circuit hose 6 is inserted into the lower part of the hose connecting portion ,24. In this embodiment the hoses 6 and 8 are thus coaxial, which is an advantage as to space in many applications.

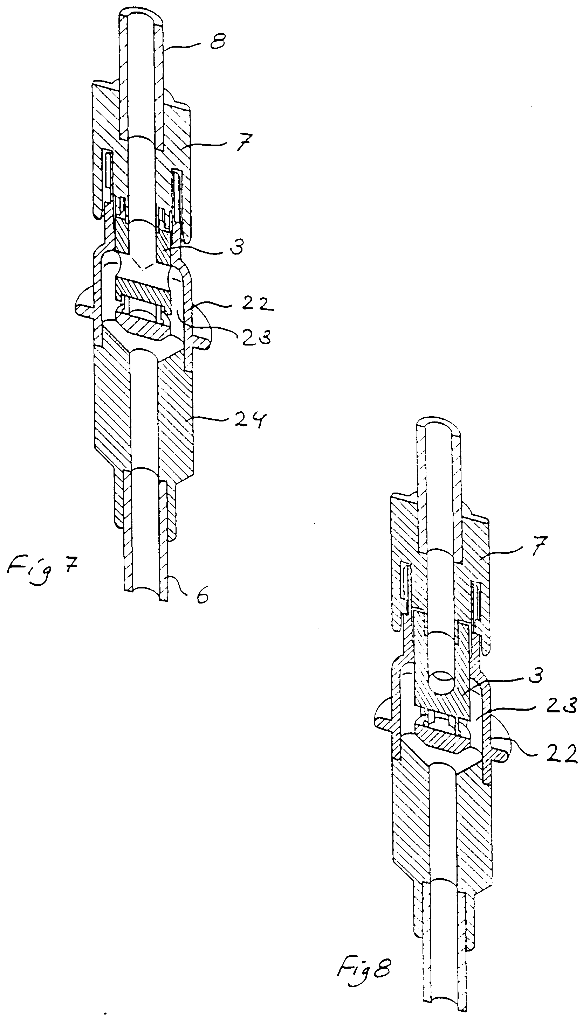

Fig. 8 shows the third embodiment with the connecting unit 7 in an inserted position and the valve body 3 in a closed position.

Figs. 9a - c show the embodiment according to Figs. 7 and 8 in an exploded sectional perspective view. In Fig. 9c an angle position lug is shown, which limits the turning position of the valve body 3. Lugs 25 are present in the coupling housing 1 as well as in the coupling housing 20. The lug 25 cooperates with stop shoulders 26 (see Fig. 3b) on the valve body. For the sake of clarity the fastening grove is not shown here.

Fig. 10 shows the coupling housing 22 in a perspective view.

The connecting device according to the invention is particularly simple to manufacture. The parts comprising the coupling device are preferably manufactured from synthetic materials. Thus it has been found suitable to manufacture the coupling housing from poly carbonate plastic and the valve body as well as the connecting unit of "Delrin" (TradeMark). Other materials may of course also be used.

Even if the invention is very suitable in connection with indtroducing different fluids from a bag reservoir, it may also be used in other medical applications.

The septum unit has only been described in connection with the first embodiment, but is of course applicable in connection with all embodiments of the invention.

The invention is not limited to the three described embodiments but only to the definitions of the following claims. Thus a different configuration of the channels may be present. The joining of the connecting unit to the coupling housing may also be achieved in any other way than by the above described bayonet joint. A joint by means of e.g. a defined thread portion is thus fully feasible. The turning movement may also be effected in another angle than the 90° mentioned in the text above.

In order to emphasize the joining and the opening position of the circuit connection, the grove 10 may in its inner part be provided with a minimal upward cut to achieve locking indication.

The fastening projection 33 may of course have another shape than the ring-shaped radially outwards directed one that is shown in the figures. Boss-like projections may e.g. come into question. The projections may also be radially inwards directed for cooperation with a radially outward directed grove which is positioned on a boss-like formation in the inner part of the coupling housing.

## Claims

1. Connecting device of synthetic material for fluid conduits for medical purposes, comprising a coupling housing (1) having at least one circuit connector (2) and a connecting unit (7) being insertable into the coupling housing and then connectable thereto by a defined turning movement, said connecting unit comprising an essentially cylindrical central extension (12) for conduit cooperation with a recess in a valve body (3), which is directly sealingly contained within the coupling housing, said connecting unit (7) and valve body (3) having means (9,15) for mutual rotational engagement, **characterized** in that the valve body (3) is provided with a central channel portion (34), which is coaxial with said turning movement, said channel portion (34) at its upper part adjoining to a ring-shaped integral seat surface (13b) within said recess for direct sealed connection to a lower surface (13a) on an integral sealing nipple (12), which comprises the extension, to achieve a smooth conduit connection, that the valve body (3) is provided with radially resilient integral fastening projections (33) for snap-in cooperation with snap-in recesses (14) within the coupling housing (1), said fastening projections (33) being arranged in a configuration so as to allow rotation of the valve body (3) with respect to the coupling housing (1) and that the means (9,15) for mutual rotational engagement are engaging means (9) which engage in engaging recess (15) in the valve body (3).

2. Device according to claim 1, **characterized** in that the valve body (3) has an inverted T-shaped channel configuration, where the horizontal portion cooperates with two circuit connectors (2) and the vertical portion with the connecting unit.

3. Device according to claim 1 or 2, **characterized** in that the circuit connectors (2) are arranged diametrically opposite to one another in the coupling housing (1).

4. Device according to claim 1, **characterized** in that the valve body (3) is provided with an L-shaped channel configuration, where the horizontal portion cooperates with the circuit connector (2) and the vertical portion with the connecting unit (7).

5. Device according to any of the previous claims, **characterized** in that the connecting unit is comprised of a septum unit (19), containing a penetratable septum (18).

6. Device according to any of the previous claims, **characterized** in that the coupling housing (1) is provided with diametrically oppositely arranged circuit nipples (5) for the connection of circuit hoses (6) to the coupling housing (1).

7. Device according to any of the previous claims, **characterized** in that the circuit connector in the coupling housing (22) is comprised of at least one peripherally arranged axial channel (23) which is parallel to the axis of the valve body (3).

8. Device according to any of the previous claims, **characterized** in that the valve body (3) comprises turning limiting stop shoulders (26) for cooperation with lugs (25) in the coupling housing.

9. Device according to any of the previous claims, **characterized** in that said engaging means (9) are comprised of axially extending arms in the connecting unit (7), which with a fit engage in axial cuts comprising the engaging recesses (15) in the valve body.

10. Device according to any of the previous claims, **characterized** in that said fastening projections (33) are arranged on an axially downward from the valve body projecting, partly cylindrical sleeve shaped portion (35) with a radius that is less than that of the envelope surface of the valve body (3).

11. Device according to claim 10, **characterized** in that said snap-in recess (14) is comprised of a groove which is formed radially outward in the bottom area in the coupling housing (1).

## Patentansprüche

1. Verbindungsvorrichtung aus Kunststoffmaterial für Fluidleitungen für medizinische Zwecke mit einsm Kupplungsgehäuse (1) mit wenigstens einem Fluidkreis-Anschluß (2) und einer Verbindungseinheit (7), die in das Kupplungsgehäuse einsetzbar und dann durch eine definierte Drehbewegung damit verbindbar ist, wobei die Verbindungseinheit eine im wesentlichen zylindrische zentrale Verlängerung (12) aufweist, die als Leitung zusammenwirkt mit einer Ausnehmung in einem Ventilkörper (3), der im Kupplungsgehäuse mit unmittelbarer Dichtung enthalten ist, wobei die Verbindungseinheit (7) und der Ventilkörper (3) Mittel (9, 15) für gegenseitigen Rotationseingriff haben, **dadurch gekennzeichnet, daß** der Ventilkörper (3) mit einem zentralen Kanalabschnitt (34) ausgebildet ist, der koaxial mit der Drehbewegung ist und an seinem oberen Teil an eine ringförmige integrale Sitzfläche (13b) in der Ausnehmung angrenzt, um eine direkte dichte Verbindung mit einer unteren Fläche (13a) an einem integralen Dichtungsnippel (12) zu bilden, der einen Fortsatz aufweist, um eine glatte Leitungsverbindung zu erreichen, daß der Ventilkörper (3) mit radial nachgiebigen integralen Haltevorsprüngen (33) für einrastendes Zusammenwirken mit Rastausnehmungen (14) im Kupplungsgehäuse (1) versehen ist, wobei die Haltevorsprünge (33) in einer solchen Konfiguration angeordnet sind, daß sie die Drehung des Ventilkörpers (3) bezüglich des Kupplungsgehäuses (1) zulassen, und daß die Mittel (9, 15) für gegenseitigen Rotationseingriff Eingriffsmittel (9) sind, welche in eine Eingriffsausnehmung (15) im Ventilkörper (3) eingreifen.

2. Vorrichtung nach Anspruch 1, **dadurch gekennzeichnet, daß** der Ventilkörper (3) eine Kanalkonfiguration in umgekehrter T-Form hat, wo der horizontale Teil mit zwei Fluidkreis-Anschlüssen (2) und der vertikale Teil mit der Verbindungseinheit zusammenwirkt.

3. Vorrichtung nach Anspruch 1 oder 2, **dadurch gekennzeichnet, daß** die Fluidkreis-Anschlüsse (2) einander diametral entgegengesetzt im Kupplungsgehäuse (1) angeordnet sind.

4. Vorrichtung nach Anspruch 1, **dadurch gekennzeichnet, daß** der Ventilkörper (3) mit einer L-förmigen Kanalkonfiguration ausgebildet ist, wo der horizontale Teil mit dem Fluidkreis-Anschluß (2) und der vertikale Teil mit der Verbindungseinheit (7) zusammenwirken.

5. Vorrichtung nach einem der vorangehenden Ansprüche, **dadurch gekennzeichnet, daß** die Verbindungseinheit aus einer Septum-Einheit (19) besteht, die ein durchdringbares Septum (18) enthält.

6. Vorrichtung nach einem der vorangehenden Ansprüche, **dadurch gekennzeichnet, daß** das Kupplungsgehäuse (1) mit diametral entgegengesetzt angeordneten Fluidkreis-Nippeln (5) zur Verbindung von Fluidkreis-Schläuchen (6) mit dem Kupplungsgehäuse (1) versehen ist.

7. Vorrichtung nach einem der vorangehenden Ansprüche, **dadurch gekennzeichnet, daß** der Fluidkreis-Anschluß im Kupplungsgehäuse (22) aus wenigestens einem peripher angeordneten axialen Kanal (23) besteht, der parallel zur Achse des Ventilkörpers (3) ist.

8. Vorrichtung nach einem der vorangehenden Ansprüche, **dadurch gekennzeichnet, daß** der Ventilkörper (3) die Drehung begrenzende Stopschultern (26) aufweist, die mit Nasen (25) im Kupplungsgehäuse zusammenwirken.

9. Vorrichtung nach einem der vorangehenden Ansprüche, **dadurch gekennzeichnet, daß** das Eingriffsmittel (9) aus axial vorstehenden Armen in der Verbindungseinheit (7) besteht, die passend in axiale Einschnitte eingreifen, welche die Eingriffs-Ausnehmungen (15) im Ventilkörper aufweisen.

10. Vorrichtung nach einem der vorangehenden Ansprüche, **dadurch gekennzeichnet, daß** die Haltevorsprünge (33) an einem axial nach unten vom Ventilkörper vorspringenden, teilweise zylindrischen manschettenförmigen Teil (35) mit einem Radius, der geringer als der der Hüllfläche des Ventilkörpers (3) ist, angeordnet sind.

11. Vorrichtung nach Anspruch 10, **dadurch gekennzeichnet, daß** die Rastausnehmung (14) aus einer Nut besteht, die radial nach außen im Bodenbereich im Kupplungsgehäuse (1) geformt ist.

## Revendications

1. Dispositif de raccordement en matière synthétique pour conduits de fluides à usage médical, comportant un boîtier d'accouplement (1) ayant au moins un connecteur de circuit (2) et une unité de raccordement (7) apte à être insérée dans le boîtier d'accouplement puis à y être raccordée par un mouvement rotatif défini, ladite unité de raccordement comportant un prolongement central essentiellement cylindrique (12) pour assurer une coopération par conduit avec un évidement prévu dans un corps de soupape (3) qui est contenue de façon directement étanche à l'intérieur du boîtier d'accouplement, ladite unité de raccordement (7) et le corps de soupape (3) ayant des moyens (9,15) pour assurer un engagement rotatif mutuel, caractérisé en ce que le corps de soupape (3) est doté d'une partie formant canal central (34) gui est coaxiale avec ledit mouvement rotatif, ladite partie formant canal (34) étant adjacente, au niveau de sa partie supérieure, à une surface de siège intégral annulaire (13b) à l'intérieur dudit évidement pour assurer un raccordement étanche direct à une surface inférieure (13a) sur un raccord d'étanchéité intégral (12), qui constitue le prolongement, pour obtenir un raccordement de conduit sans discontinuité, en ce que le corps de soupape (3) est doté de saillies de fixation intégrales résilientes dans le sens radial (33) pour assurer une coopération à encliquetage avec des évidements à encliquetage (14) à l'intérieur du boîtier d'accouplement (1), lesdites saillies de fixation (33) étant disposées suivant une configuration de nature à permettre la rotation du corps de soupape (3) par rapport au boîtier d'accouplement (1), et en ce que les moyens (9, 15) prévus pour un engagement rotatif mutuel sont des moyens d'engagement (9) qui s'engagent dans évidement d'engagement (15) du corps de soupape (3).

2. Dispositif selon la revendication 1, caractérisé en ce que le corps de soupape (3) présente une configuration de canal en forme de T inversé, la partie horizontale coopérant avec deux connecteurs de circuit (2) et la partie verticale coopérant avec l'unité de raccordement.

3. Dispositif selon la revendication 1 ou 2 caractérisé en ce que les connecteurs de circuit (2) sont disposés de manière à se trouver diamétralement opposés l'un à l'autre dans le boîtier d'accouplement (1).

4. Dispositif selon la revendication 1, caractérisé en ce que le corps de soupape (3) est doté d'une configuration de canal en forme de L, la partie horizontale coopérant avec le connecteur de circuit (2) et la partie verticale coopérant avec l'unité de raccordement (7).

5. Dispositif selon l'une quelconque des revendications précédentes, caractérisé en ce que l'unité de raccordement se compose d une unité à septum (19), contenant un septum pénétrable (18).

6. Dispositif selon l'une quelconque des revendications précédentes, caractérisé en ce que le boîtier d'accouplement (1) est doté de raccords de circuit (5) disposés de manière à être diamétralement opposés pour le raccordement de tuyaux de circuit (6) au boîtier d'accouplement (1).

7. Dispositif selon l'une quelconque des revendications précédentes, caractérisé en ce que le connecteur de circuit du boîtier d'accouplement (22) se compose d'au moins un canal axial disposé périphériquement (23) qui est parallèle à l'axe du corps de soupape (3).

8. Dispositif selon l'une quelconque des revendications précédentes, caractérisé en ce que le corps de soupape (3) comporte des épaulements (26) de butée de limitation de rotation destinés à coopérer avec des pattes (25) du boîtier d'accouplement.

9. Dispositif selon l'une quelconque des revendications précédentes, caractérisé en ce que lesdits moyens d'engagement (9) se composent de bras s'étendant axialement dans l'unité de raccordement (7) qui s'engagent dans des découpes axiales constituant les évidements d'engagement (15) du corps de soupape.

10. Dispositif selon l'une quelconque des revendications précédentes, caractérisé en ce que lesdites saillies de fixation (33) sont disposées sur une partie (35) en forme de manchon partiellement cylindrique faisant saillie axialement vers le bas à partir du corps de soupape, ayant un rayon qui est inférieur à celui de la surface d'enveloppe du corps de soupape (3).

11. Dispositif selon la revendication 10, caractérisé en ce que ledit évidement (14) à encliquetage se compose d'une rainure qui est formée radialement à l'extérieur dans la zone de fond dans le boîtier d'accouplement (1).
